# NEW EUROPEAN PATENT SPECIFICATION

(11) **EP 0 536 939 B2**
(45) Date of publication and mention of the opposition decision: **10.03.1999**
(45) Mention of the grant of the patent: 20.03.1996
(21) Application number: 92308919.7
(22) Date of filing: 30.09.1992
(51) Int. Cl.: C07C 213/02

(54) **Process for the production of aminopolyols**
Verfahren zur Herstellung von Aminopolyolen
Procédé pour la préparation des aminopolyols

(30) Priority: 08.10.1991 GB 9121279
(43) Date of publication of application: 14.04.1993
(73) Proprietor: CERESTAR HOLDING BV, NL-4550 AA Sas Van Gent (NL)
(72) Inventor: Beck, Roland Herwig, Dr., B-1160 Bruxelles (BE); Kalff, Norbert Johannes, W-4150 Krefeld 12 (DE); Röper, Harald, W. W., Dr., B-1180 Bruxelles (BE)
(74) Representative: Wilkinson, Stephen John

(56) References cited:
- EP-A- 0 022 529
- EP-A- 0 238 961
- DE-A- 3 625 931
- US-A- 2 016 962
- US-A- 2 197 540
- US-A- 3 187 047
- Morris Freifelder, Catalytic Hydrogenation in organic synthesis procedures and commentary, John Wiley & Sons, New York-Chichester-Brisbane-Toronto (1978), pp. 90 - 93
- Morris Freifelder, Practical catalytic hydrogenation, John Wiley & Sons, New York-Chichester-Brisbane-Toronto (1971), pp. 333-336
- Houben-Weyl. Methoden der Organischen Chemie, Band XI/1 (1957), pp. 606-611
- J. Am. Chem. Soc. 72, 5416 (1950)
- Holleman-Wiberg, Lehrbuch der Anorganischen Chemie, Walter de Gruyter-Verlag, Berlin (1955), p. 229

## Description

The present invention relates to the production of aminopolyols, in particular to the production of aminopolyols by the reductive amination of a saccharide.

The amination of saccharides by ammonia or an aliphatic amine having a replaceable amino hydrogen atom has been known for many years, see for example US patent 2 016 962 issued in 1935. The process described in this patent is carried out at an elevated pressure of more than 15 atmospheres, at a temperature between 50°C and 200°C and in the presence of a base metal hydrogenation catalyst, nickel usually being preferred. The ammonia or amine and the hydrogen are preferably in considerable excess to minimise side reactions and the catalyst is preferably mounted on a solid support.

The aminopolyols are commercially interesting as chemical intermediates and in particular for their potential use in the manufacture of surface active compounds. It has been our experience however that when using the process described above, problems can arise in obtaining the desired reaction selectivity. We have now found however that significant improvements in selectivity can be obtained if there is added to the reaction medium a compound which, under the reaction conditions is capable of giving rise to NH₄⁺.

According to the present invention there is provided a process for the production of an aminopolyol which comprises reacting a monosaccharide or a reducing oligosaccharide with ammonia or with an alphatic amine having a replaceable amino hydrogen atom in the presence of hydrogen and a base metal catalyst characterised in that the reaction is carried out in the presence of from 0.1 to 5.0% by weight based on the weight of the monosaccharide or reducing oligosaccharide of a compound not being water which, under the reaction conditions, is capable of providing NH₄⁺ without poisoning the catalyst and in that the monosaccharide is an aldose or ketose containing 5 or 6 carbon atoms in the molecule.

The compound which is capable of providing NH₄⁺ under the reaction conditions may be an organic acid particularly a carboxylic acid, or a derivative of an organic acid eg. carbon dioxide, a carboxylic acid anhydride, a carboxylic acid lactone or a salt particularly an ammonium salt or the salt of a metal such as nickel which is reduced in the reaction medium by the hydrogen present to give metallic nickel and NH₄⁺. An inorganic mineral acid on the contrary eg. hydrochloric or sulphuric acid although capable of producing the necessary NH₄⁺ is not suitable for use in the process because the coproduct Cl⁻ and SO₄⁻⁻ are both poisons for base metal catalysts such as nickel and cobalt.

US patent No. 2 197 540 describes a process in which an aldose sugar is reacted with ammonia to give a crystalline product referred to, for example, as glucose-ammonia. The process comprises heating the sugar such as glucose under anhydrous conditions with ammonia in the presence of an acid catalyst. Both organic and inorganic acids are said in the patent to be effective and as examples there are quoted hydrochloric, acetic, monochloracetic, trichloracetic, benzoic, citric and tartaric acids. The process of the US patent is not however a reductive amination since no hydrogen is present and no distinction is made between the use of inorganic and organic acid catalysts.

There is also a patent publication, DT-PS 36 09 978 which describes a two stage process of reductive amination of a specific group of hydroxy-compounds R¹-CO-CH(OH)-R² where R¹ and R² are CH₂CH or H. It is said in this publication that the addition of an ammonium salt, specifically ammonium chloride or ammonium acetate, can be advantageous but no indication is given of the nature of the advantage and there is no Example in the patent in which an ammonium salt is added to the reaction medium. We have found in our process that although ammonium acetate gives the desired improvement in selectivity the addition of ammonium chloride does not but produces instead a reduction in selectivity.

The compound which is capable of providing NH₄⁺ under the reaction conditions is added in an amount which is 0.1 to 5.0% by weight of the saccharide, more preferably 1 to 4% by weight. Although there is a large number of carboxylic acids which may be used either as acid or salt it is most convenient to use a C₂ to C₁₀ alkanoic acid or a similar acid substituted by a simple substituent eg. acetic acid, propionic acid, capric acid, lactic acid or glycine. Di- and polycarboxylic acids are also suitable for use in the process according to the invention and again the acid may contain other substituents, eg. citric acid. It is usually preferred that the carboxylic additive to the process should be the acid or ammonium salt since we have found that the greatest improvement in selectivity is obtained in this manner.

The base metal catalyst which is used in the process may be chosen from those conventially employed in reductive amination processes. Of these, nickel and to a lesser extent cobalt, are most widely used more often as Raney nickel or Raney cobalt ie the solid metal catalyst in finely divided form. We have found that in the process according to the present invention Raney cobalt is to be preferred over Raney nickel. The amount of base metal catalyst is suitably 4 to 20% by weight preferably 5 to 10% by weight based on the saccharide.

The monosaccharide is an aldose or a ketose having five or six carbon atoms in the molecule eg. xylose, glucose, galactose, mannose and fructose. Glucose and dextrose are particularly preferred because of their commercial availability. A reducing oligosaccharide is an oligosaccharide in which an aldehyde position (or hemiacetal) is exposed and suitable oligosaccharides for use in the process of the invention are the disaccharides, particularly maltose and lactose, trisaccharides eg. maltotroise and tetra- and higher saccharides, especially the mixture of oligosaccharides obtained by the acid or enzymatic hydrolysis of starch.

The saccharide is reacted with ammonia or with an aliphatic amine having a replaceable amino hydrogen atom, ie with a primary or secondary amine. Suitable amines include simple alkylamines containing up to 18 carbon atoms such as monomethylamine, diethylamine, monobutylamine and dioctylamine and aliphatic amines in which the aliphatic radical is substituted eg by an aromatic or heterocyclic group as in benzylamine or by a group such as hydroxy or amino. The molar ratio of saccharide to ammonia, when the latter is reactant, preferably lies between 1:5 and 1:10 more preferaby between 1:6 and 1:7 whereas when an amine is reactant the ratio is preferably between 0.5:1 and 1.5:1, more preferaby between 0.8:1 and 1.2:1.

The amination reaction may be carried out in a solvent which may be water or a hydroxylic solvent such as a lower alkanol eg. methanol. The amount of the solvent is suitably in the range 0 to 50% by weight preferably 0 to 20% by weight of the total reaction composition.

The hydrogen pressure during the process is preferably held in the range of 50 to 250 bar, more preferably 90 to 120 bar and the temperature is preferably maintained less than 50°C, more preferably less than 40°C until the initial reaction of the saccharide with the ammonia or amine has taken place. Subsequently, the temperature is slowly raised at, for example, 0.1 to 1.0°C per minute particularly 0.2 to 0.5°C per minute until the hydrogenation reaction commences at, for example, between 40° and 80°C. The temperature of the reaction mixture is then increased to 80°C to 120°C preferably 85° to 95°C and held at this temperature until hydrogen uptake ceases when the pressure is released and the reaction mixture allowed to cool down preparatory to removing the catalyst eg. by filtration and recovering the aminopolyol eg. by crystallisation. Alternatively and preferably, after cooling and release of the pressure but before filtration, the reaction mixture is submitted to a post-hydrogenation in order to complete the reaction and facilitate subsequent catalyst removal. Other techniques which may be applied in isolating the aminopolyol include the removal of volatile components by distillation, decolourisation of the reaction product and/or spray-drying of the aminopolyol.

An alternative, and preferable, operating procedure is to premix the base metal catalyst, the compound capable of providing NH₄⁺ and the liquid ammonia or aliphatic amine. The hydrogen pressure is then raised to value in the range 50 to 250 bar, more preferably in the range 90 to 120 bar and the temperature raised to between 80°C to 120°C preferaby 85°C to 95°C. A solution of the monosaccharide or reducing oligosaccharide is then slowly added over a period of, for example, 1 to 4 hours. After all the solution has been added the temperature is held at 85°C to 95°C until hydrogen uptake ceases. The hydrogen pressure is then released and the aminopolyol recovered as described above.

The invention will now be further illustrated by reference to the following examples.

### Example 1

A 10 litre capacity autoclave was purged twice with nitrogen at 10 bar pressure before being charged with 3603 g of a 70% by weight aqueous glucose solution, 144 g Raney cobalt (5.7% by weight based on glucose dry substance) and 63 g ammonium acetate (2.5% by weight based on glucose dry substance). The autoclave was then flushed out once with nitrogen (10 bar) and twice with hydrogen (20 bar) before stirring at 1200 rpm was started under a hydrogen pressure of 5 bar. Liquefied, water-free ammonia was next introduced into the cooled autoclave at such a rate as to maintain the temperature of the autoclave contents at below 40°C. The addition time for 1669 g ammonia was approximately one hour and on completion of the addition the cooling of the autoclave was discontinued and the hydrogen pressure increased to 80 bar. The hydrogenation reaction was initiated by increasing the temperature in the autoclave at a rate of 0.3°C/min. and at 57°C a slight drop in pressure of the hydrogen indicated the commencement of reaction. The hydrogen pressure was then increased to 100 bar and held constant until the temperature reached 95°C. The progress of the reaction was followed by the uptake of hydrogen (alternatively by determining the amount of glucose remaining in solution).

When hydrogen uptake ceased, the autoclave was cooled to 85°C when the pressure was released and the hydrogen and residual ammonia allowed to evaporate. On cessation of the evaporation about 5% of ammonia remained in the reaction mixture so the autoclave was repressured with 90 bar hydrogen and reheated to 90°C for a further 30 mins. Finally, the pressure was released, the temperature reduced to 60°C and the catalyst removed by filtration under nitrogen at a pressure of 5 to 10 bar.

The reaction mixture after filtration contained 60% by weight solids which had the following composition as determined by high pressure liquid chromatography (HPLC)
83.4 % by weight 1-aminosorbitol
3.1 % by weight 2-aminosorbitol
3.5 % by weight 2-aminomannitol
2.2 % by weight sorbitol
2.6 % by weight disorbitylamine
3.8 % by weight degradation products

The 1-aminosorbitol was recovered by crystallisation from aqueous methanol.

### Example 2

Example 1 was repeated but the catalyst and amount of ammonium acetate added were varied. The variations and the quantities of 1-aminosorbitol produced were as follows.

| Example | 2(a) | 2(b) | 2(c) |
|---|---|---|---|
| Catalyst | Raney cobalt | Raney nickel | Raney nickel |
| Amount of Ammonium Acetate (% by weight glucose) | none | 2.5 | none |
| Temperature at commencement of reaction | 60°C | 55°C | 60°C |
| Selectivity (1-aminosorbitol as percentage by weight | 60.6% | 64.0% | 45.7% |
| of solid reaction product) | | | |

### Example 3

The procedure described in Example 1 was repeated using Raney nickel as catalyst and varying the carboxylic acid/carboxylate added. The selectivities obtained were as follows :-

| | | |
|---|---|---|
| 3(a) (comparative) | no carboxylic acid/carboxylate | 45.7 % |
| 3(b) | ammonium acetate | 64.0 % |
| 3(c) | ammonium carbonate | 65.7 % |
| 3(d) | citric acid | 66.9 % |
| 3(e) (comparative) | ammonium chloride | 44.2 % |
| 3(f) (comparative) | ammonium sulphate | 43.8 % |
| 3(g) | nickel acetate | 58.0 % |

### Example 4

The procedure of Example 1 was followed using (a) Raney cobalt and (b) Raney nickel and varying the amount of ammonium acetate added. The selectivities obtained are shown below.

| Amount of Ammonium Acetate Added (% by weight glucose) | Selectivities | |
|---|---|---|
| | (a) | (b) |
| None | 60.6 % | 45.7 % |
| 0.25 | 71.0% | 56.5% |
| 0.80 | 76.5% | 62.0% |
| 2.5 | 83.4% | 64.0% |

### Example 5

A 10 litre capacity autoclave was purged twice with nitrogen at 10 bar pressure before being charged with 144 g Raney cobalt and 63 g ammonium acetate. The autoclave was then flushed out once with nitrogen (10 bar) and twice with hydrogen (20 bar). Liquified, water free ammonia was next introduced into the autoclave, the hydrogen pressure raised to 100 bar and the autoclave brought to 85°C. 4204 g of a 60% by weight aqueous glucose solution was then dosed into the autoclave over a time period of 2.5 hours. 0.5 hour after all the glucose solution had been added, the temperature of the autoclave was raised to 95°C and held at this temperature for a further 0.5 hour.

When hydrogen uptake ceased, the autoclave was cooled to 85°C, when the pressure was released and the hydrogen and the residual ammonia allowed to evaporate. At the end of the evaporation about 5% of ammonia remained in the reaction mixture so that the autoclave was repressured with 80 bar hydrogen and reheated to 90°C for a further 30 min. Finally the pressure was released, the temperature reduced to 60°C and the catalyst removed by filtration under nitrogen at a pressure of 5 to 10 bar.

The reaction mixture after filtration contained about 55% by weight solids which had the following composition as determined by high pressure liquid chromatography (HPLC)
88.6 % by weight 1-aminosorbitol
0.9 % by weight 2-aminosorbitol
0.9 % by weight 2-aminomannitol
2.6 % by weight sorbitol
1.4 % by weight disorbityl amine
5.6 % by weight degradation products

## Claims

1. A process for the production of an aminopolyol which comprises reacting a monosaccharide or reducing oligosaccharide with ammonia or with an aliphatic amine having a replaceable amino hydrogen atom in the presence of hydrogen and a base metal catalyst characterised in that reaction is carried out in the presence of from 0.1 to 5.0% by weight based on the weight of the monosaccharide or reducing oligosaccharide of a compound not being water which, under the reaction conditions, is capable of providing NH₄⁺ without poisoning the catalyst and in that the monosaccharide is an aldose or ketose containing 5 or 6 carbon atoms in the molecule.

2. A process according to either claim 1, further characterised in that the monosaccharide is glucose.

3. A process according to either claim 1 or claim 2, wherein the aliphatic amine having a replaceable amino hydrogen atom is an alkylamine containing up to 18 carbon atoms or an aliphatic amine substituted by an aromatic or heterocyclic group or by an hydroxy or amino group.

4. A process according to any one of the preceding claims, wherein the molar ratio of saccharide to ammonia lies between 1:5 and 1:10 or the ratio of the saccharide to amine lies between 0.5:1 and 1.5:1.

5. A process according to any one of the preceding claims, wherein the compound which under the reaction conditions is capable of providing NH₄⁺ in the reaction medium is carbon dioxide, ammonium carbonate or is a carboxylic acid or the ammonium salt of such an acid e.g., a C₂ to C₁₀ alkanoic acid or a substituted C₂ to C₁₀ alkanoic acid.

6. A process according to any one of the preceding claims, wherein the compound which, under the reaction conditions is capable of providing NH₄⁺, is added in an amount which is 1 to 4% by weight of the saccharide.

7. A process according to any one of the preceding claims, wherein the base metal catalyst is nickel or cobalt, preferably Raney nickel or Raney cobalt.

8. A process according to any one of the preceding claims, wherein Raney nickel or Raney cobalt catalyst is premixed with liquid ammonia and with a carboxylic acid, ammonium carboxylate or ammonium carbonate, the mixture heated to a temperature of 80° to 120°C at a hydrogen pressure of 50 to 250 bar and a solution of an aldose e.g., glucose or a disaccharide e.g., maltose slowly added over a period of 1 to 4 hours.

9. A process according to any one of the preceding claims, wherein the reaction is carried out in water or other hydroxylic solvent e.g., methanol.

## Patentansprüche

1. Verfahren zur Herstellung eines Aminopolyols, umfassend Umsetzen eines Monosaccharids oder reduzierenden Oligosaccharids mit Ammoniak oder mit einem aliphatischen Amin, das ein ersetzbares Aminowasserstoffatom aufweist, in Gegenwart von Wasserstoff und einem basischen Metallkatalysator, dadurch gekennzeichnet, daß die Umsetzung in Gegenwart von 0,1 bis 5,0 Gew.-%, bezogen auf das Gewicht des Monosaccharids oder reduzierenden Oligosaccharids, einer von Wasser verschiedenen Verbindung ausgeführt wird, die unter den Umsetzungsbedingungen in der Lage ist, NH₄⁺ bereitzustellen, ohne den Katalysator zu vergiften und daß das Monosaccharid eine 5 oder 6 Kohlenstoffatome in dem Molekül enthaltende Aldose oder Ketose ist.

2. Verfahren nach Anspruch 1, weiterhin dadurch gekennzeichnet, daß das Monosaccharid Glucose ist.

3. Verfahren nach Anspruch 1 oder Anspruch 2, wobei das aliphatische Amin mit einem ersetzbaren Aminowasserstoffatom ein Alkylamin, das bis zu 18 Kohlenstoffatome enthält oder ein aliphatisches Amin, substituiert mit einer aromatischen oder heterocyclischen Gruppe oder mit einer Hydroxy- oder Aminogruppe, ist.

4. Verfahren nach einem der vorangehenden Ansprüche, wobei das Molverhältnis des Saccharids zu Ammoniak zwischen 1:5 und 1:10 liegt oder das Verhältnis des Saccharids zum Amin zwischen 0,5:1 und 1,5:1 liegt.

5. Verfahren nach einem der vorangehenden Ansprüche, wobei die Verbindung, welche unter Umsetzungsbedingungen in der Lage ist, NH₄⁺ in dem Reaktionsmedium bereitzustellen, Kohlendioxid, Ammoniumcarbonat ist oder eine Carbonsäure oder das Ammoniumsalz einer solchen Säure, beispielsweise einer C₂- bis C₁₀-Alkansäure oder einer substituierten C₂- bis C₁₀-Alkansäure, ist.

6. Verfahren nach einem der vorangehenden Ansprüche, wobei die Verbindung, die unter den Umsetzungsbedingungen in der Lage ist, NH₄⁺ bereitzustellen, in einer Menge zugegeben wird, die 1 bis 4 Gew.-% des Saccharids ist.

7. Verfahren nach einem der vorangehenden Ansprüche, wobei der basische Metallkatalysator Nickel oder Cobalt, vorzugsweise Raney-Nickel oder Raney-Cobalt, ist.

8. Verfahren nach einem der vorangehenden Ansprüche, wobei der Raney-Nickel- oder der Raney-Cobalt-Katalysator mit flüssigem Ammoniak und mit einer Carbonsäure, Ammoniumcarboxylat oder Ammoniumcarbonat vorvermischt wird, das Gemisch auf eine Temperatur von 80° bis 120°C bei einem Wasserstoffdruck von 50 bis 250 bar erhitzt wird und eine Lösung einer Aldose, beispielsweise Glucose oder eines Disaccharids, beispielsweise Maltose, langsam über einen Zeitraum von 1 bis 4 Stunden zugegeben wird.

9. Verfahren nach einem der vorangehenden Ansprüche, wobei die Umsetzung in Wasser oder anderem Hydroxylgruppen enthaltenden Lösungsmittel, beispielsweise Methanol, ausgeführt wird.

## Revendications

1. Procédé pour la production d'un aminopolyol, qui comprend la réaction d'un monosaccharide ou d'un oligosaccharide réducteur avec l'ammoniac ou avec une amine aliphatique comportant un atome d'hydrogène de fonction amino remplaçable, en présence d'hydrogène et d'un catalyseur formé par un métal de base, procédé caractérisé en ce que la réaction est conduite en présence de 0,1 à 5,0 % en poids, par rapport au poids du monosaccharide ou de l'oligosaccharide réducteur, d'un composé autre que l'eau qui, dans les conditions de la réaction, est capable de fournir NH₄⁺ sans empoisonner le catalyseur, et en ce que le monosaccharide est un aldose ou un cétose contenant 5 ou 6 atomes de carbone dans sa molécule.

2. Procédé selon la revendication 1, caractérisé en outre en ce que le monosaccharide est le glucose.

3. Procédé selon l'une des revendications 1 ou 2, dans lequel l'amine aliphatique comportant un atome d'hydrogène de fonction amino remplaçable est une alkylamine contenant jusqu'à 18 atomes de carbone ou est une amine aliphatique substituée par un groupe aromatique ou hétérocyclique ou par un groupe hydroxyle ou amino.

4. Procédé selon l'une quelconque des revendications précédentes, dans lequel le rapport molaire du saccharide à l'ammoniac se situe entre 1:5 et 1:10, ou le rapport du saccharide à l'amine se situe entre 0,5:1 et 1,5:1.

5. Procédé selon l'une quelconque des revendications précédentes, dans lequel le composé qui, dans les conditions de réaction, est capable de fournir NH₄⁺ dans le milieu de réaction est le dioxyde de carbone, le carbonate d'ammonium ou est un acide carboxylique ou le sel d'ammonium d'un tel acide, par exemple un acide alcanoïque en C₂ à C₁₀ ou un acide alcanoïque substitué en C₂ à C₁₀.

6. Procédé selon l'une quelconque des revendications précédentes, dans lequel le composé qui, dans les conditions de réaction, est capable de fournir NH₄⁺ est ajouté en une quantité qui est de 1 à 4 % en poids du saccharide.

7. Procédé selon l'une quelconque des revendications précédentes, dans lequel le catalyseur formé par du métal de base est le nickel ou le cobalt, de préférence le nickel de Raney ou le cobalt de Raney.

8. Procédé selon l'une quelconque des revendications précédentes, dans lequel le catalyseur constitué par ou comportant du nickel de Raney ou du cobalt de Raney est prémélangé avec l'ammoniac liquide et avec un acide carboxylique, avec du carboxylate d'ammonium ou du carbonate d'ammonium, le mélange est chauffé jusqu'à une température de 80° à 120 °C sous une pression d'hydrogène de 50 à 250 bar, et l'on ajoute lentement, en une période de 1 à 4 h, une solution d'un aldose, par exemple du glucose, ou d'un disaccharide, par exemple le maltose.

9. Procédé selon l'une quelconque des revendications précédentes, dans lequel on effectue la réaction dans de l'eau ou dans un autre solvant hydroxylique, par exemple le méthanol.
